# EUROPEAN PATENT SPECIFICATION

(11) **EP 3 742 163 B1**
(45) Date of publication and mention of the grant of the patent: **28.09.2022**
(21) Application number: 19175451.4
(22) Date of filing: 20.05.2019
(51) Int. Cl.: G01N 33/483, A61B 5/00, G01N 3/08, G01N 3/04

(54) **METHOD FOR MEASURING HAIR COMPRESSION AND SOFTNESS**
VERFAHREN ZUR MESSUNG DER HAARKOMPRESSION UND -WEICHHEIT
PROCÉDÉ DE MESURE DE COMPRESSION ET DE DOUCEUR DE CHEVEUX

(43) Date of publication of application: 25.11.2020
(73) Proprietor: L'OREAL, 75008 Paris (FR)
(72) Inventor: GALLIANO, Anthony, 92110 Clichy (FR); SEWRAJ, Poonam, 92110 Clichy (FR)
(74) Representative: L'Oreal

(56) References cited:
- US-A- 4 635 654
- US-A1- 2011 217 256

## Description

### Field of the invention

The present invention relates to a method measuring hair compression and softness, in order to evaluate and/or analyze typological characteristics of the hair, notably mechanical characteristics, for example, to establish a diagnosis.

The present invention also relates to a device particularly intended for measuring hair compression and softness.

### Background of the invention

Hair damage is characterized by dull looking, brittle fibers which tend to break easily and have increased porosity. In addition, cuticle cells tend to be uplifted indicating a hair damage with the presence of split ends.

The damage is mostly caused by three types of constraints: mechanical, chemical and environmental. For example, too frequent or excessive brushing or combing can cause damages, split ends and uplifted cuticle cells. Likewise, over-vigorous chemical treatments such as perming, dying, bleaching or too alkaline shampoos can also cause damages by hair protein hydrolysis and oxidation of cysteine linkages.

To better understand in which extent hair has been damaged by such factors or treatments, or to evaluate how damages have been repaired, objective measurements of hair properties can be performed. These measurements are mechanical such as, for example, measuring the hair's resistance to breakage. Some measurements can be chemical, such as, for example, measuring the hair's solubility in selected media.

These experiments often require specific instrumentation which is suitable for carrying out measurements on Caucasian hair but not suitable for carrying out measurements on African hair.

In particular, African people generally have frizzy hair that are difficult to manage, to comb or to groove without causing any breakages. In order to limit the breakages or the hair loss, African people use various hair products for example for hydration, softness and better feel.

Some methods and tools are available for the measurement of softness or smoothness of hair having a curl type I to a curl type IV. Nevertheless, these methods are mostly suitable for rather straight hair (Aqualon test, 3 points bending), meaning for hair having a curl type of III or less. Moreover, there are also some tests available in some industries to measure compression force:
- The wool industry uses a specific method to measure the softness in wool,
- The food industry uses a specific method to evaluate the texture of creams or confectionaries,
- The bedding industry uses a specific method to evaluate the comfort of beds and mattress.

These criteria of softness, suppleness and smoothness can also be evaluated with sensorial tests, not on global scale but only through a qualitative comparison versus a standard for European or Asian hair. However, there is no tool that can measure the softness on very curly hair.

To overcome this lack, hairdressers or even consumers, carry out a manual evaluation by pressing, scrunching and gliding a swatch of hair between the thumb and the forefinger. This way, they feel the resistance of the swatch to compression and its ability to recover its original form when released, which are taken as the basis of estimation. Of course, this measurement lacks accuracy.

The document US 4 635 654 A discloses a device for measuring hair compression and softness having a longitudinal axis. It comprises a body, a piston that is movable relative to the body and that co-operates therewith to define at least partially a storage chamber of variable volume for containing a swatch of hair, a thread member arranged to thread the swatch into the body.

The document US 2011/217256 together with "MTT 1 75 - Miniature Tensile Tester", 30 October 201 9 (201 9-1 0-30), XP055637392, Retrieved from the Internet: URI-:http://www.dproscientific.com/assets/dia-stron-mttl 75-brochure.pdf [retrieved on 201 9-1 0-30] also discloses the same features as US 4 635 654 A.

There is a need to provide a sensitive, direct and objective measurement method for determining hair compression and softness for frizzy hair, particularly for African hair.

There is a need to provide such a method for in vitro objective and quantitative evaluations that can serve for all type of hair curliness (from type I to type VIII) but that is even more suitable for hair with a high degree of curliness (greater to type V).

There is a need to provide such a method for evaluating the level of softness according to a level of curliness, in order to assess the immediate and long lasting performances of cosmetic products.

There is a need to provide such a method for processing the output data, in view of a better understanding of the link between hair compression and hair softness.

There is a need to provide such a method for comparing the performance of different products, such as softening products or stiffening products.

There is a need to provide such a method for an improved communication with hairdressers, consumers or brands concerning the quality of hair.

There is a need to provide such a method that:
- is easy to employ,
- uses a small amount of hair,
- requires a short time,
- appeals to uncomplicated equipment, allowing the method to be practiced in almost all laboratories in the world.

### Summary of the invention

According to a first of its aspects, the invention provides a device for measuring hair compression and softness having a longitudinal axis X comprising:
a body and a piston that is movable relative to the body and that co-operates therewith to define at least partially a storage chamber of variable volume for containing a swatch of hair,
a thread member arranged to thread the swatch into the body, the body comprising a channel for the passage of a wire connected to the swatch of hair, the channel being arranged to thread the swatch into the body,a system for driving in translation the piston into the body at a controlled speed and over a controlled distance of the piston, from a first position in which the swatch of hair is in a rest state to a second position in which the swatch of hair is in a crushing state,
a system for recording the piston force needed to crush the swatch up to the crushing state.

The device according to the invention is of high significance to quantify with a high precision, the texture, the body and the softness of a frizzy swatch of hair.

Thanks to the device according to the invention, it is now possible to obtain intrinsic characteristics of a hair product by evaluating several swatches.

This device according to the invention allows to evaluate the benefit of a hair product during a treatment and the lastingness of the product after the treatment.

The device according to invention is easy to use and quite cheap.

The device according to invention allows to transform communication with consumers.

The device according to the invention is key in understanding frizzy hair.

According to a second aspect, the invention relates to a method for measuring hair compression and softness, comprising:
- Providing a device according to anyone of the preceding claims,
- Introducing a swatch of hair in the storage chamber of the body,
- Threading the swatch into the body using the thread member,
- Driving in translation the piston into the body, from a first position in which the swatch of hair is in a rest state to a second position in which the swatch of hair is in a crushing state,
- Recording parameters needed to crush the swatch from the rest state up to the crushing state and, eventually, to let the swatch relax from the crushing.

In accordance with preferred embodiments, the device preferably communicates with an external computer network, which may be by wired (e.g., USB) connection but preferably is wireless (WiFi, Bluetooth, etc.). In preferred embodiments, the device communicates wirelessly to an external network and also to a companion device such as a smartphone, tablet, notebook computer or other computing device.

### Preferred embodiments

According to advantageous aspects of the invention, the device comprises one or several of the following features taken in isolation or in any technically possible combinations:
- The system for driving in translation the piston into the body also drives the piston away from the swatch at a controlled speed and over a controlled distance. According to the length of the swatch, the piston stroke can be specified according to the length of the swatch.
- The body is located on a support member and the support member defines with the body, the storage chamber.
- With a support member, it becomes easy to replace the body, notably as the body can be inserted and maintained in the support member by any assembly, such as screwing, force fitting or snap-in.
- The body comprises two cylindrical containers. Such a structure makes it possible to test in vivo a small amount of hair, such as swatch weighing less than 1 gram and also a large amount of hair, such a swatch weighting between 1gram to 4gram.
- It comprises a system for calculating the height of the swatch measured along the longitudinal axis X as the piston moves away from the swatch.
- Thanks to this structure of the thread member, the swatch of hair is introduced into the chamber without any deformation.
- It comprises a processing unit configured to calculate at least of an average force of compression of the swatch, a maximum force of compression of the swatch, an energy or a work of compression and a relaxation time of the swatch.
- It comprises a network interface for communicating with an external server via an Internet connection or with Bluetooth, Wi-Fi or WiMAX or any other mode exchange of data, by wired or wireless or wired and non-wired channel mixed.

According to advantageous aspects of the invention, the process comprises one or several of the following features taken in isolation or in any technically possible combinations:
- It comprises calculating at least one of an average force of compression of the swatch, a maximum force of compression of the swatch, an energy or a work of compression and a relaxation time of the swatch.
- It comprises displaying on a screen the results of the calculation.
- The device is calibrated to minimize the margin of error on the calculated results.

### Detailed description

The invention will be understood better from reading the following detailed description of non-limiting exemplary embodiments thereof and from studying the appended schematic and partial drawings, in which:
Fig.1 depicts a side view of a device according to the invention before a measurement, the body being set with a narrow diameter cylinder,
Fig. 2 depicts a face view of a device according to the invention during the crushing of a swatch, the body being set with a large diameter cylinder,
Fig. 3 depicts a swatch of hair before a measurement with the device according to the invention,
Fig. 4 depicts a face view in cross section of a device according to the invention before a measurement, the body being set with a narrow diameter cylinder,
Fig. 5 depicts a side view in cross section of a device according to the invention, before a measurement, the body being set with a large diameter cylinder,
Fig. 6 depicts the average values of total work W (Joule) calculated with the device according to the invention on swatches of hair, before the application of treatment (on the right) and after the application of a treatment (on the left).

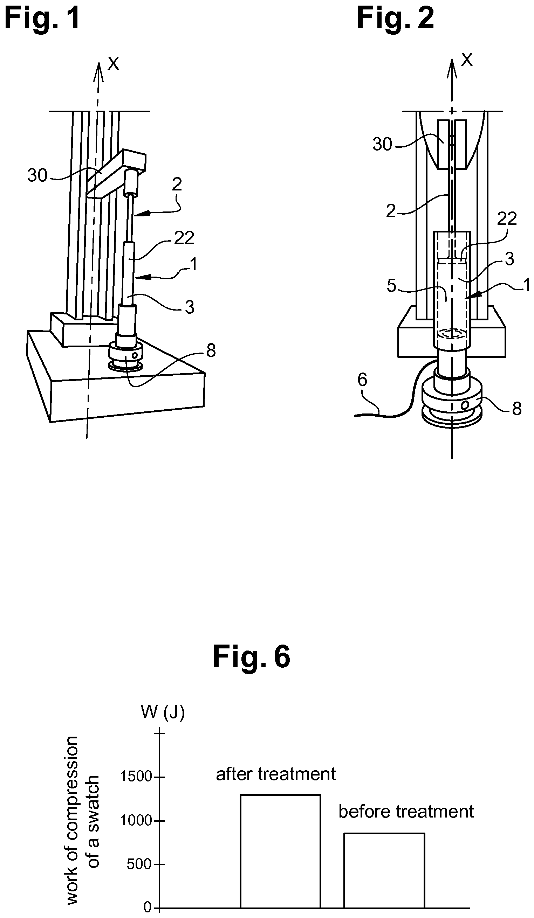

FIG. 1 shows an exemplary device according to the invention having a longitudinal axis X. The device may comprise a body 1 forming a cylindrical receptacle for the swatch of hair 5 and a support 8 on which the body 1 can be secured by screwing.

The body 1 may include a main cylindrical wall 12 and a bottom wall 13 which may be provided with an opening 14 that enables the support 8 to be inserted through.

The support 8 may comprise: an assembly skirt 7 that enables the body 1 to be screwed on the neck 9; a substantially cylindrical wall 10 forming a housing 11 for receiving a part of the swatch; and a channel 4 extending inclined outside the housing 11 through the assembly skirt. The wall 10 may have a cross-section that may be circular, elliptical or oval. Preferably, the cross section of the wall 10 is circular. In the exemplary embodiment, the body 1 may be screwed on the neck 9. However, in other exemplary embodiments, the body 1 may be secured in some other way, for example, by screw-fastening, snap-fastening or crimping.

The body 1 includes a piston 2 that moves along an inside surface of the wall 12. Together with the body 1 and the support member 8, the piston 2 may form a storage chamber 3 of variable volume located beneath the piston 2. The piston 2 is defined by a planar front face 22 coming into abutment against the swatch of hair 5 to crush it and by a rear face 23 receiving the rod 24. The body 1 might be provided with an air exhaust hole (not shown) to make the movement of the piston more fluid.

The piston 2 may be driven by any drive mechanism 30 that moves under the user's action on a control element such as a pusher, a thumbwheel or an electronic input. The drive mechanism 30 can be operated by a motor under the control of an electronic control unit (not shown). It might be able to move the piston 2 between a first position in which the swatch 5 of hair is in a rest state to a second position in which the swatch 5 of hair is in a crushing state. In the example, the driven mechanism is Ametek LS1 ^{®} marketed by Lloyd.

A force sensor (not represented) might be mounted on the device according to the invention, in order to capture the force of the piston during a measurement. In a preferred embodiment, the longitudinal axis of the piston 2, the longitudinal axis of the body 1 and the longitudinal axis of the force sensor are identical, in order to reduce frictions between the piston 2 and the body 1, as it could cause measurement errors.

The body 1 might be made of a transparent material, such as glass, PMMA, PVC or polycarbonate, to give the user the opportunity to follow the course of the piston 2 and to measure its displacement into the body 1.

On Fig. 2, a wire 6 is attached to the swatch 5 of hair and goes through the channel 4 to be pulled outward by the user, until it comes to an optimal position to start the measurements. The wire 6 allows the swatch 5 of hair to be threaded into the body 1 without any pressure and deformation, notably on the top of the swatch 5. The arrow depicts the direction of the movement of the piston.

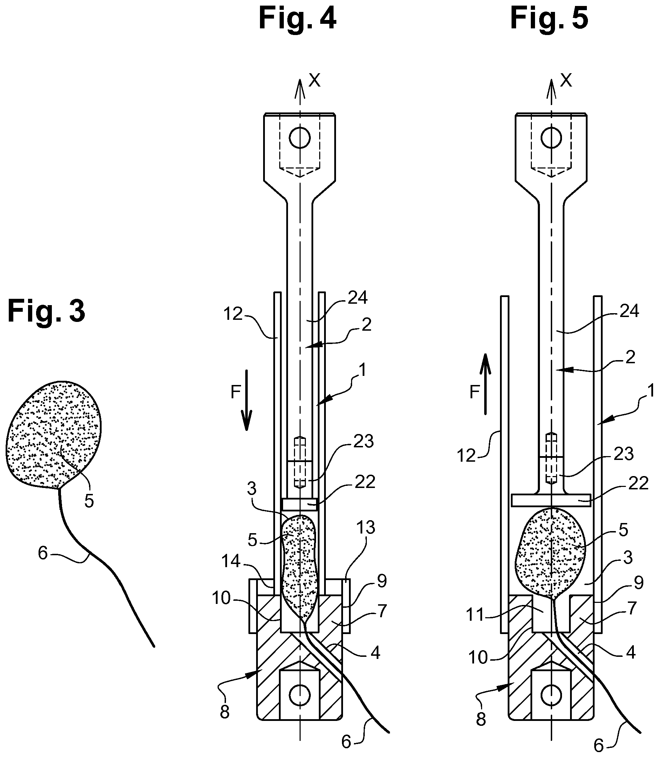

Fig. 3 shows a swatch 5 with a wire 6 attached to it for insertion into the body 1, thanks to an opening available on the support of the body 1, such as a channel 6.

Fig. 4 and 5 illustrate the method for measuring hair compression and softness according to the invention.

The measurement can comprise:
- Pulling the swatch 5 into the body 1 with the help of the wire 6 until it is secured into the chamber 3,
- Moving the piston 2 down (down arrow on Fig. 4) along the wall of the body 1 up to a referenced height, preferably without hitting the top of the swatch 5,
- Further moving the piston 2 down on the swatch 5 to crush it at a controlled speed, up to a final predetermined compression height,
- Moving back (up arrow on Fig. 5) the piston 2 to its initial position at a controlled speed,
- Recording the force necessary to crush the swatch 5 as a function of the position of the piston 2 along the longitudinal axis X,
- Following the relaxation of the swatch 5 by measuring its height according to its recovering time.

Fig. 6 illustrates the use of the device according to the invention, to evaluate the softness, body and texture of a swatch of hair before and after the application of a cosmetic product.

The bar on the right represents the average value of a computed total work (W) of compression of a swatch before (right) and after (left) an application of a cosmetic product. The average value is calculated from four swatches of hair of curliness type VII. The average total work is computed after 5 cycles of compression.

The total works increases after the application of the hair product, showing a higher texture and body performance of the hair after the application of the product. On the opposite, a decrease of the compression force would show a higher softening performance of the hair for softer hair, easier to manage than before the application of the product.

Although the present invention herein is described with reference to particular exemplary embodiments, it is to be understood that these embodiments are merely illustrative of the principles and applications of the present invention. It is therefore to be understood that numerous modifications may be made to the illustrative embodiments and that other arrangements may be deduced without departing from the scope of the present invention.

## Claims

1. A device for measuring hair compression and softness having a longitudinal axis (X), **characterized in that** it comprises:
a body (1) and a piston (2) that is movable relative to the body and that co-operates therewith to define at least partially a storage chamber of variable volume for containing a swatch (5) of hair, **characterized by** :
a thread member arranged to thread the swatch into the body, the body (1) configured to encase a swatch (5) of hair, the body comprising a channel (4) for the passage of a wire (6) connected to the swatch (5) of hair, and the device comprises
a system for driving in translation the piston (2) into the body at a controlled speed and over a controlled distance of the piston (2), from a first position in which the swatch (5) of hair is in a rest state to a second position in which the swatch of hair is in a crushing state,
a system for recording the piston (2) force needed to crush the swatch (5) up to the crushing state.

2. A device according to claim 1, **characterized in that** the system for driving in translation the piston (2) into the body (1) also drives the piston (2) away from the swatch (5) at a controlled speed and over a controlled distance.

3. A device according to anyone of the preceding claims, **characterized in that** the body (1) is located on a support member (8) and the support member (8) defines with the body (1), the storage chamber.

4. A device according to anyone of the preceding claims, **characterized in that** the body (1) comprises two cylindrical containers.

5. A device according to anyone of the preceding claims, **characterized in that** it comprises a system for calculating the height of the swatch (5) measured along the longitudinal axis (X) as the piston (2) moves away from the swatch (5).

6. A device according to anyone of the preceding claims, **characterized in that** it comprises a processing unit configured to calculate at least one of an average force of compression of the swatch (5), a maximum force of compression of the swatch (5), an energy or a work of compression and a relaxation time of the swatch (5).

7. A device according to anyone of the preceding claims, **characterized in that** it comprises a network interface for communicating with an external server via an Internet connection or with Bluetooth, Wi-Fi or WiMAX or any other mode exchange of data, by wired or wireless or wired and non-wired channel (4) mixed.

8. A method for measuring hair compression and softness, **characterized in that** it comprises:
(i) Providing a device according to anyone of the preceding claims,
(ii) Introducing a swatch (5) of hair in the storage chamber (3) of the body (1),
(iii) Threading the swatch (5) into the body (1) using the thread member,
(iv) Driving in translation the piston (2) into the body (1), from a first position in which the swatch (5) of hair is in a rest state to a second position in which the swatch (5) of hair is in a crushing state,
(v) Recording parameters needed to crush the swatch (5) from the rest state up to the crushing state and, eventually, to let the swatch (5) relax from the crushing.

9. A method according to claim 8, **characterized in that** it comprises calculating at least pone of an average force of compression of the swatch (5), a maximum force of compression of the swatch (5), an energy or a work of compression and a relaxation time of the swatch (5).

10. A method according to anyone of the claim 8 or 9, **characterized in that** it comprises displaying on a screen the results of the calculation according to claim 7.

11. A method according to anyone of the claims 8 to 10, **characterized in that** the device is calibrated to minimize the margin of error on the calculated results.

## Patentansprüche

1. Vorrichtung zur Messung der Haarkompression und Weichheit, welche eine Längsachse (X) aufweist, **dadurch gekennzeichnet, dass** sie umfasst:
einen Körper (1) und einen Kolben (2), der relativ zu dem Körper beweglich ist und damit zusammenwirkt, um mindestens teilweise eine Aufbewahrungskammer mit variablem Volumen zu definieren, um eine Probe (5) aus Haar zu enthalten,
**gekennzeichnet durch**:
ein Fädelelement, das vorgesehen ist, um die Probe in den Körper zu fädeln,
den Körper (1), der ausgestaltet ist, um eine Probe (5) des Haars zu umhüllen,
wobei der Körper einen Kanal (4) zum Durchgang eines Drahts (6) umfasst, der mit der Probe (5) des Haars verbunden ist, und wobei die Vorrichtung umfasst:
ein System, um den Kolben (2) translatorisch mit einer kontrollierten Geschwindigkeit und über eine kontrollierte Distanz des Kolbens (2) aus einer ersten Position, in der die Probe (5) des Haars sich in einem Ruhezustand befindet, in eine zweite Position, in der die Probe des Haars sich in einem Stauchzustand befindet, in den Körper zu treiben,
ein System zum Aufzeichnen der Kraft des Kolbens (2), die benötigt wird, um die Probe (5) bis zu dem Stauchzustand zu stauchen.

2. Vorrichtung nach Anspruch 1, **dadurch gekennzeichnet, dass** das System zum translatorischen Treiben des Kolbens (2) in den Körper (1) auch den Kolben (2) mit einer kontrollierten Geschwindigkeit und über eine kontrollierte Distanz von der Probe (5) weg treibt.

3. Vorrichtung nach einem der vorhergehenden Ansprüche, **dadurch gekennzeichnet, dass** der Körper (1) sich auf einem Trägerelement (8) befindet und das Trägerelement (8) mit dem Körper (1) die Aufbewahrungskammer definiert.

4. Vorrichtung nach einem der vorhergehenden Ansprüche, **dadurch gekennzeichnet, dass** der Körper (1) zwei zylindrische Behälter umfasst.

5. Vorrichtung nach einem der vorhergehenden Ansprüche, **dadurch gekennzeichnet, dass** sie ein System zur Berechnung der Höhe der Probe (5) umfasst, gemessen entlang der Längsachse (X), wenn sich der Kolben (2) von der Probe (5) weg bewegt.

6. Vorrichtung nach einem der vorhergehenden Ansprüche, **dadurch gekennzeichnet, dass** sie eine Verarbeitungseinheit umfasst, die ausgestaltet ist, um mindestens eine von einer durchschnittlichen Kompressionskraft der Probe (5), einer maximalen Kompressionskraft der Probe (5), einer Energie oder einer Arbeit der Kompression und einer Relaxationszeit der Probe (5) zu berechnen.

7. Vorrichtung nach einem der vorhergehenden Ansprüche, **dadurch gekennzeichnet, dass** sie eine Netzwerkschnittstelle zum Kommunizieren mit einem externen Server über eine Internetverbindung oder mit Bluetooth, Wi-Fi oder WiMAX oder jedwedem anderen Datenaustauschmodus mittels verdrahtetem oder drahtlosem oder gemischt verdrahtetem und nichtverdrahtetem Kanal (4) umfasst.

8. Verfahren zur Messung der Haarkompression und Weichheit, **dadurch gekennzeichnet, dass** es umfasst:
(i) Bereitstellen einer Vorrichtung nach einem der vorhergehenden Ansprüche,
(ii) Einbringen einer Probe (5) von Haar in die Aufbewahrungskammer (3) des Körpers (1),
(iii) Fädeln der Probe (5) in den Körper (1) unter Verwendung des Fädelelements,
(iv) translatorisches Treiben des Kolbens (2) in den Körper (1) aus einer ersten Position, in der die Probe (5) des Haars sich in einem Ruhezustand befindet, in eine zweite Position, in der die Probe (5) des Haars sich in einem Stauchzustand befindet,
(v) Aufzeichnen von Parametern, die erforderlich sind, um die Probe (5) aus dem Ruhezustand bis in den Stauchzustand zu stauchen, und schließlich Relaxierenlassen der Probe (5) von dem Stauchvorgang.

9. Verfahren nach Anspruch 8, **dadurch gekennzeichnet, dass** es Berechnen von mindestens einer von einer durchschnittlichen Kompressionskraft der Probe (5), einer maximalen Kompressionskraft der Probe (5), einer Energie oder einer Arbeit der Kompression und einer Relaxationszeit der Probe (5) umfasst.

10. Verfahren nach einem der Ansprüche 8 oder 9, **dadurch gekennzeichnet, dass** es Anzeigen der Ergebnisse der Berechnung gemäß Anspruch 7 auf einem Bildschirm umfasst.

11. Verfahren nach einem der Ansprüche 8 bis 10, **dadurch gekennzeichnet, dass** die Vorrichtung kalibriert ist, um die Fehlerspanne der berechneten Ergebnisse zu minimieren.

## Revendications

1. Dispositif servant à mesurer la compression et la souplesse de cheveux ayant un axe longitudinal (X), **caractérisé en ce qu'**il comprend :
un corps (1) et un piston (2) qui est mobile par rapport au corps et qui coopère avec lui pour définir au moins partiellement une chambre de stockage de volume variable destinée à contenir un échantillon (5) de cheveux, **caractérisé par** :
un élément fileté agencé pour enfiler l'échantillon à l'intérieur du corps,
le corps (1) étant configuré pour enfermer un échantillon (5) de cheveux,
le corps comprenant un canal (4) pour le passage d'un fil (6) relié à l'échantillon (5) de cheveux, et le dispositif comprenant
un système destiné à entraîner en translation le piston (2) à l'intérieur du corps à une vitesse contrôlée et sur une distance contrôlée du piston (2), depuis une première position dans laquelle l'échantillon (5) de cheveux est dans un état de repos jusqu'à une deuxième position dans laquelle l'échantillon de cheveux est dans un état d'écrasement,
un système destiné à enregistrer la force du piston (2) nécessaire pour écraser l'échantillon (5) jusqu'à l'état d'écrasement.

2. Dispositif selon la revendication 1, **caractérisé en ce que** le système destiné à entraîner en translation le piston (2) à l'intérieur du corps (1) entraîne également le piston (2) loin de l'échantillon (5) à une vitesse contrôlée et sur une distance contrôlée.

3. Dispositif selon l'une quelconque des revendications précédentes, **caractérisé en ce que** le corps (1) est situé sur un élément de support (8) et l'élément de support (8) définit avec le corps (1) la chambre de stockage.

4. Dispositif selon l'une quelconque des revendications précédentes, **caractérisé en ce que** le corps (1) comprend deux récipients cylindriques.

5. Dispositif selon l'une quelconque des revendications précédentes, **caractérisé en ce qu'**il comprend un système destiné à calculer la hauteur de l'échantillon (5) mesurée le long de l'axe longitudinal (X) lorsque le piston (2) s'éloigne de l'échantillon (5).

6. Dispositif selon l'une quelconque des revendications précédentes, **caractérisé en ce qu'**il comprend une unité de traitement configurée pour calculer au moins un paramètre parmi une force de compression moyenne de l'échantillon (5), une force de compression maximale de l'échantillon (5), une énergie ou un travail de compression et un temps de relaxation de l'échantillon (5) .

7. Dispositif selon l'une quelconque des revendications précédentes, **caractérisé en ce qu'**il comprend une interface réseau destinée à communiquer avec un serveur externe par le biais d'une connexion Internet ou par Bluetooth, Wi-Fi ou WiMax ou tout autre mode d'échange de données, par une voie (4) filaire ou sans fil ou mixte filaire et non filaire.

8. Procédé servant à mesurer la compression et la souplesse de cheveux, **caractérisé en ce qu'**il comprend :
(i) l'obtention d'un dispositif selon l'une quelconque des revendications précédentes,
(ii) l'introduction d'un échantillon (5) de cheveux dans la chambre de stockage (3) du corps (1),
(iii) l'enfilage de l'échantillon (5) à l'intérieur du corps (1) au moyen de l'élément fileté,
(iv) l'entraînement en translation du piston (2) à l'intérieur du corps (1), depuis une première position dans laquelle l'échantillon (5) de cheveux est dans un état de repos jusqu'à une deuxième position dans laquelle l'échantillon (5) de cheveux est dans un état d'écrasement,
(v) l'enregistrement de paramètres nécessaires pour écraser l'échantillon (5) depuis l'état de repos jusqu'à l'état d'écrasement et, éventuellement, pour laisser l'échantillon (5) récupérer de l'écrasement.

9. Procédé selon la revendication 8, **caractérisé en ce qu'**il comprend le calcul d'au moins un paramètre parmi une force de compression moyenne de l'échantillon (5), une force de compression maximale de l'échantillon (5), une énergie ou un travail de compression et un temps de relaxation de l'échantillon (5).

10. Procédé selon l'une quelconque des revendications 8 et 9, **caractérisé en ce qu'**il comprend l'affichage sur un écran des résultats du calcul selon la revendication 7.

11. Procédé selon l'une quelconque des revendications 8 à 10, **caractérisé en ce que** le dispositif est étalonné pour minimiser la marge d'erreur sur les résultats calculés.
